# EUROPEAN PATENT APPLICATION

(11) **EP 1 439 172 A1**
(43) Date of publication of application: **21.07.2004**
(21) Application number: 02779105.2
(22) Date of filing: 24.09.2002
(51) Int. Cl.: C07D 307/70, A61K 31/341, A61P 33/02

(54) **METHOD OF OBTAINING 2-(2-NITROVINYL)-FURAN AND THE USE THEREOF AS A COCCIDIOSTATIC**

(30) Priority: 28.09.2001 CU 21901
(71) Applicant: Centro De Bioactivos Quimicos (CBQ), Santa Clara, Villa Clara 54830 (CU)
(72) Inventor: OLAZ BAL MANSO, E. E. Calle 1ra Edif. 3,A3, Santa Clara Villa Clara 50300 (CU); CASTANEDO CANCIO, Nilo R. Maceo 463 Sur, Santa Clara Villa Clara 50100 (CU); GAIT N PLACERES, Teofilo E. Toscano No. 828, Santa Clara Villa Clara 50100 (CU); RODR GUEZ NEGR N, Zenaida, Placetas Villa Clara 52800 (CU); SERRANO PEREZ, Héctor,Zoilo Estrada Palma No. 204, Santa Clara Villa Clara 50100 (CU); DIAZ MOLINA, Maria I. Carretera Central y Circunv., SantaClara Vill a Clara 50400 (CU); CALVO ALONSO, Amalia, Maria; Carretera de Sagua, Santa Clara Villa Clara 50300 (CU); GONZALEZ HERNANDEZ, Orlando, Caibarien, Villa Clara 52610 (CU)
(74) Representative: Winckels, Johannes Hubertus F.
(86) International application number: PCT/CU2002/000007
(87) International publication number: WO 2003/051858

(57) **Abstract**

This invention is related with a procedure for the obtention and purification of 2-(2-nitrovynil)-furan in a step of reaction starting from furfural and nitro methane using isobuthylamine as a catalyst and activated coal as an adsorbent, achieving a product with pharmaceutical quality.

This technology appreciably reduces the environmental impact through eliminations of the gassy residual from the system through absorption in water.

The pharmaceutical composition proposed in this invention contains 2- (2-nitrovynil)-furan and presents a potent action against *Coccidia at* low concentrations.

## Description

### Technical sector

This invention is related with the animal and human sector particularly with a product having a potent anticoccidial activity. It has been shown very satisfactory results in experimentation and industrial exploitation animals used as a source of foods.

### Previous technique

It has been reported, in scientific articles and in information patents that furan products, particularly nitrofuran compounds, show action against *Coccidia.* In spite of the fact that these compounds are still available in the market, their consumption have been diminishing because they have toxic properties with the position of the nitro group in the carbon 5 of the furan ring. These products are furazolidone. (FUROBAC) and nitrofurazone, among others.

(FUROBAC http://terra.com.mx/-rcamacho/ges/ges-furo.html), Environmental Mutagenesis 5: 745-765 (1983), Com.Reg. 19.03.92 (L73), Com.Reg. 15.10.93 (L 264)

The obtention of 2- (2-nitrovynil)-furan as a chemical entity has been reported earlier only at laboratory level, and these are not antecedents of their use as human or animal medication. Its synthesis has not been reported in any case as active principle with pharmaceutical quality.

The purity of the compound has not been reported in the literature describing its synthesis at the laboratory level, which prevent from evaluating if it fulfills those requirements needed to be used as medication. Neither the studies on the reproducibility of the results in the syntheses , nor the reactions to scales different from those at molar fractions are reported, as can be appreciated in the following references:
1.A.L. MNOAZHOYANAN, Edit. ARMENIAN, Erevan 1960.
2.- O. MOLDENHAUER, W.IRION, D.MOSTALGLIO, R.PFLUGER, H.DOSER, A.583,37 (1953), [pat]. German 918.148 (1954)[ CA.52 .15590h (1958)].
3.- B.PRIEB, BER 181362 (885)
4.- L.BOUVEAULT, A.WAHL, BULL.SOC.CHIM[3] 29.525 (1903).
5.- M.OKAWARA.J.CHEM.SOC.JAPAN 56.90 (1953) C.A 49,4618d (1955).
6.- H.SAIKACHI H. HOSHIDA, YAKUGAKU, 78.917 (1958) C.A52 21136b (1958)

There are also some patents where 2-(2-nitrovynil)-furan is used as part of synergic mixtures for industrial antimicrobial applications, but they do not describe aspects related with its synthesis. Among others they are the patents of the North American Company Great Lakes Chemical Co. (US 5,416,107; US 5,416,108; US 5,358,963; US 5,246,963 and WO 94/02017).

### "Popularization" of the Invention

This invention is based on a simple compound of furan base, 2-(2-nitrovynil)-furan, which presents a nitro group in its structure, but not in the position 5 of the ring but in the double exociclic bond. It has been corroborated through predictive and experimental methods that this variation in the position of the nitro group in this family modifies favorably both its pharmacological and toxicological properties.

This invention also includes a new procedure for obtaining 2-(2-nitrovynil)-furan, which consists of reacting the nitromethane and the furfural in presence of ethanol in molar reactions 1: 1 to 1: 2.5, during 3 to 5 hours, in a range of temperatures between 110 and 130°C, using from 1 to 2.5 ml of isobutyl amine (IBA) per mol of furfural, with a speed of agitation between 100 and 400 rpm.

The condenser of the reaction equipment is connected to absorption traps containing water to absorb nitrous gases to avoid the environmental contamination.

The reaction mixture obtained in those conditions undergoes a process of "cooling" between -15 +10°C. Then the solid is separated by means of a process of centrifugation or filtration in vacuum.

The raw product becomes purified dissolving it in 4 to 10 volumes of ethanol at a temperature between 40 and 65°C during 10 to 30 minutes, with 5 to 25% of activated coal. Later it is filtered on activated coal and allowed to precipitate of a temperature between -15 and 10°C. Then, the pure product is filtered dried off in a stove at vacuum during 1 to 3.5 hours at a temperature that oscillates between 20 and 50oC and stored at a temperature between 10 and 20oC.

By means of the procedure of this invention, which is executed at the moment at pilot plant scale, the 2-(2-nitrovynil)-furan is obtained in form of yellow crystals with a fusion temperature of 74-75 Celsius degrees, with high yields and purity that surpass the 99%, which permit the use of this active principle in the pharmaceutical industry. The structural analysis of this product was performed with the use of all modern spectroscopic techniques.

The concentration of 2-(2-nitrovynil)-furan in the pharmaceutical composition, for its use in the treatment of *Coccidia* in human and veterinary medicine, oscillates in a range between 2 and 80 ppm.

Toxicological studies, necessary to perform studies of effectiveness of the product, showed that the Half Lethal Dose (LD50) for oral route of 2-(2-nitrovynil)-furan using several solvents, was 1.02 g/kg of body mass for mature Leghorn hens and 171.91 mg/kg of body mass for females and males Sprague Dawley rats.

*In vitro* mutagenesis studies were carried out using the micronucleus assay with blockade of cytokinesis with and without metabolic activation, as well as the sister chromatide exchange assay with and without metabolic activation. It was are carried out a theoretical study assisted by computer for the prognosis of the mutagenic activity. In all those 2-(2-nitrovynil)-furan did not shown mutagenic activity.

In order to evaluate the effectiveness against *Coccidia* from several animal species, artificial infection experiments of *Eimeria tenella* in poultry, as well as in high naturally infected chicken with this protozoa were carried out. The effect against the intestinal *Coccidia* of artificially and naturally infected rabbits was also evaluated the last in conditions of production.

Examples of the realization of this invention are fallow described.

### EXAMPLE 1:

The synthesis of 2-(2-nitrovynil)-furan was carried out by means of the procedure of the invention, varying several parameters inside the reported ranges.

Table 1 shows the degrees of purity obtained in the synthesis of 2-(2-nitrovynil)-furan carried out in 6 liters reactors, under different assayed conditions.

**Table**

| Num. synthesis | Molar rel. Furf/NM | Quantity of IBA (ml/Mol) of G-0 | Reaction Temp.. (°C) | Time (hours) | Activated charcoal (%/g of raw G-0) | Alcohol volume (ml/g of raw G-0) | % Purity (*) |
|---|---|---|---|---|---|---|---|
| 1 | 1-2.0 | 1.0 | 110 | 3.0 | 5 | 4 | 99.59 |
| 2 | 1-2.2 | 1.5 | 115 | 3.5 | 7.5 | 6 | 99.35 |
| 3 | 1-2.5 | 1.2 | 125 | 5.0 | 6 | 6 | 99.81 |
| 4 | 1-2.3 | 1.6 | 140 | 3.0 | 8 | 8 | 99.09 |
| 5 | 1-2.4 | 2.3 | 110 | 3.5 | 10 | 10 | 99.75 |
| 6 | 1-2.1 | 1.4 | 120 | 4.0 | 7 | 7 | 99.61 |

| | | | | | | | |
|---|---|---|---|---|---|---|---|
| (*) The determination of the purity percent was carried out by means of HPLC. | | | | | | | |

### EXAMPLE 2.

To carried out the assay of the action of the synthesized product, the 2- (2-nitrovynil)-furan, it was first dissolved first in an alcoholic solution at 3% and later in water, using several concentrations (2, 4, 8 µg/ml) of the alcohol-water solution , as is shown Table 2.

One day old, male, free of *Coccidia,* F1 fatten chicks were used. These birds were maintained in an appropriate condition, free of *Coccidia* until two weeks of age. For feeding, it was used the same food used to fatten chickens but free of anticoccididals.. 5 groups of 12 poultry with similar weight were formed which received an inoculation of 1 x 10⁵ oocysts of *Eimeria tenella* by oral route, using a esophagic canula. The treatment began when chicks had 15 days of age, 24 hours before receiving the inoculation, during a period of 9 days.

To evaluate the effectiveness of the product the gain of weight ( % referred to the initial weight ) and the death number approaches were used in each tratment.

The results are shown in Table 2.

**Table 2.**

| Treatment | Dose µg/mL | Weight gain in % during 9 days | Mortality |
|---|---|---|---|
| 1 | 2 | 78.79 | 1/12 |
| 2 | 4 | 86.78 | 0/12 |
| 3 | 8 | 63.95 | 0/12 |
| 4 | Inoculated | 65.17 | 5/12 |
| 5 | without inoculated | 93.27 | 0/12 |

As it can be observed, satisfactory results were obtained at a concentration of 4 µg/ml, where there were no deaths and the weight gain was very similar to the control group, without inoculating.

### EXAMPLE 3

The 2-(2-nitrovynil)-furan, in a formulation similar to that described in the example 2, was applied, in the drinking water twice a day (8 am and 12 m) for 5 days, to 2000 Leghorn egg-laying hens of 56-84 days of age, infected naturally with *E. tenella.* Two houses highly infected by caecal coccidiosis with 2000 birds each were there was an average of 119 deaths per house in 5 days were selected. The diagnoses was made by the presence of bloody diarrheas , the observation of typical lesions in the blind intestine and abundant presence of parasite oocysts in the caecal content. Infected birds in a house was left as nontreated control and another no infected as neither infected nor treated control having the same range of age.

To evaluate the effectiveness of the product the approach described in the example 2 was used, except that as weight control. Except that as weight control 10 birds of each pen were randomly selected and weighed at the beginning and the end of the treatment. The obtained results are shown in table 3.

| Treatment | Doses µg/mL | Difference of weight in % during 5 days | Mortality in 5 days |
|---|---|---|---|
| 1 | 4 | 0 | 15/2000 |
| 2 | Affected no treated | -17 | 115/2000 |
| 3 | No affected, no treated | 2 | 9/2000 |

It can be observed that for the dose of 4µg/ ml there was a remarkable decrease of mortality without any weight loss when compared with the affected no treated group.

### EXAMPLE 4

The 2-(2-nitrovynil)-furan, in a formulation similar to that described in the example 2, was dissolved in rabbits' drinking water and administered daily *at libitum* for 5 days. Two experiments (I, II), each one with 9 rabbits, were carried out. Each experiment had 3 treatments of 3 animals each one. Among treatments an infected no treated group and a group neither infected nor treated were considered.. In the 1^{st} experiment each rabbit received 61 x 10³ those of the 2^{nd} experiment 116 x 10³ oocysts of intestinal Coccidias. The present species were *E. perforans* 38%, *E*. *magna* 30%, *E. media* 22% and *E. exigua* 10%, with a 97.3% of sporulation. The initial and final weight difference and the deaths of animals after 7 days treatment was used as evaluation criteria.

The obtained results are shown in table 4 and 5:

**Table 4:**

| EXPERIMENT I. INOCULACION OF RABBITS WITH 61 X 10³ OOCYSTS OF INTESTINAL EIMERIA. | | | |
|---|---|---|---|
| Treatmens | Dose µg/mL | Weight gain in % during 7 days | Mortality |
| 1 | 4 | 4 | 4 0/3 |
| 2 | Inoculated without treatment | -4 | 0/3 |
| 3 | No inoculated, no treated | 16 | 0/3 |

| Chart 5: EXPERIMENT II . INOCULACION OF RABBITS WITH 116 x 10³ OOCYSTS OF INTESTINAL EIMERIA. | | | |
|---|---|---|---|
| 1 | 4 | 3 | 0/3 |
| 2 | Inoculated without treatment | -5 | 1/3 |
| 3 | No inoculated, no treated | 16 | 0/3 |

After inoculating 61 x 10³ oocysts there was no mortality at the dose of 4 µg/mL and there was difference with the inoculated without treatment weight group in gain. When 116 x 10³ ocysts were inoculated with the dose of 4 µg/ml there were no deaths and also weight gain was present. Already at this concentration there were deaths in the inoculated without treatment.

### EXAMPLE 5

The 2-(2-nitrovynil)-furan, in a formulation similar to that described in the example 2, was dissolved in rabbit's drinking water and administered daily *at* *libitum* for 5 days at concentration of 4 µg/ml. It were treated 1 246 newborn, young and adult rabbits of the Chinchilla, New White Zelanda and New Red Zelanda species belonging to 3 rabbit companies. The species of *Coccidia* present were *E. perforans* 34%, *E. magna* 32%, *E. media* 23% and *E. exigua* 11%. The amount of oocysts per gram of feces before and after 7 days of the treatment was determined to 20% of all treated animals. The difference between the number of oocysts before and after 7 days of treatment expressed as percentage of oocyst reduction, was taken as efficacy criterium.

The obtained results are shown in table 6.

| Company | Number of animals | Reduction in % |
|---|---|---|
| 1 | 426 | 90 |
| 2 | 320 | 99 |
| 3 | 500 | 90 |
| Total | 1246 | mean = 97 |
| | | |

From the results obtained in the experiment, it could be assumed that in a relatively number of animals there was an average reduction of oocyst of 97%. In fact, in a company it was of a 99%, which is a very satisfactory result.

## Claims

1. A process for obtaining 2-(2-nitrovinyl)-furan **characterized by** the following steps:
reacting nitromethane, previously contacted with a primary amine as a catalyst, in a mixture of polar solvents, during a period of time of from 1 to 2.5 hours at a temperature between 110 and 130 °C;
adding furfural in a furfural-nitromethane molar ratio between 1:1 and 1:2.5 at the same temperature, using an agitation speed between 100 and 400 rpm and reflux conditions during a period of time of from 3 to 5 hours;
allowing the reaction mixture to cool between -15 and 10 °C and separating the crude material thus obtained by solid-liquid separation methods;
purifying the crude material with activated carbon and polar solvents at a temperature between 40 and 65 °C, with a contact time between 10 and 30 minutes;
separating the activated carbon;
cooling the purified solution between -15 and 10 °C and recovering the crystals by centrifugation of filtration under vacuum;
drying the end product at a temperature between 20 and 55 °C for a time period of from 1 to 3.5 hours.

2. A process for obtaining 2-(2-nitrovinyl)-furan according to claim 1, wherein the mixture of solvents used in the reaction between the nitromethane and the primary amine preferably are alcohols.

3. A process for obtaining 2-(2-nitrovinyl)-furan according to claim 1, wherein the primary amine used as a catalyst preferably is isobutyl amine.

4. A process for obtaining 2-(2-nitrovinyl)-furan according to claim 1, wherein the solid-liquid separation is performed preferably through centrifugation or vacuum filtration.

5. A process for obtaining 2-(2-nitrovinyl)-furan according to claim 1, wherein the polar solvent used in the purification of the crude material preferably is ethanol.

6. A process for obtaining 2-(2-nitrovinyl)-furan according to claim 1, wherein the separation of the activated carbon from the solution containing the active principle is effected preferably through centrifugation or vacuum filtration.

7. 2-(2-Nitrovinyl)-furan obtained by the process of claims 1-6 with a purity of at least 98%.

8. 2-(2-Nitrovinyl)-furan according to claims 7 with a purity of 99% or more.

9. 2-(2-Nitrovinyl)-furan with a purity of 99% or more.

10. A pharmaceutical composition **characterized by** containing 2-(2-nitrovinyl)-furan as an active principle.

11. Use of 2-(2-nitrovinyl)-furan as a medicament.

12. Use of 2-(2-nitrovinyl)-furan for the treatment of coccidiosis.
